# EUROPEAN PATENT APPLICATION

(11) **EP 1 604 697 A1**
(43) Date of publication of application: **14.12.2005**
(21) Application number: 04013671.5
(22) Date of filing: 09.06.2004
(51) Int. Cl.: A61L 31/16, A61F 2/06

(54) **Implantable device**

(71) Applicant: J.A.C.C. GmbH, 81679 München (DE)
(72) Inventor: Frohwitter, Bernhard, 81679 München (DE)
(74) Representative: Tomlinson, Edward James

(57) **Abstract**

The present invention provides an implantable device (10) as a delivery device for at least one therapeutic agent being composed of at least one type of base material comprising at least two types of reservoirs (14,16) for at least one therapeutic agent whereby each type of reservoir independently provides identical or different release rates for the at least one therapeutic agent.

## Description

### TECHNICAL FIELD

The present invention generally relates to implantable devices and therapeutic methods involving intravenous or surgical introduction of devices that are implantable into a patient, either human or non-human, such as the introduction into a region of the body such as a passage or blood vessel or other lumen or directly into soft tissue or bone.

### BACKGROUND OF THE INVENTION

In the past, various medical devices have been developed to treat a variety of medical conditions by introducing an implantable medical device partly or completely into the vascular system, esophagus, trachea, colon, biliary tract, urinary tract, pancreas, uterus or other location within a human or animal patient.

As an example, many treatments of the vascular system typically comprise the introduction of a device such as stents, catheters, cannulae, balloons or the like. Unfortunately, however, when such a device is introduced through the vascular system until positioned at the desired location, the blood vessel walls can be disturbed or even injured. Thrombosis often results at the injured site thereby causing stenosis/occlusion of the blood vessel.

Furthermore, if the medical device is positioned within the lumen of a body portion for an extended period of time, a thrombus often forms on the device itself, again causing stenosis/occlusion. As a result, the patient is placed at risk of a variety of complications, including stroke, heart attack and pulmonary embolism.

Another reason why which blood vessels undergo stenosis is through disease. For example, the most common reason causing stenosis is atherosclerosis. Atherosclerosis is a process in which deposits of fatty substances, cholesterol, cellular waste products, calcium and other substances build up in the inner lining of an artery. Plaques can grow large enough to significantly reduce the blood's flow through an artery (ischemia) The most dangerous damage, however, occurs when they become fragile and rupture. Plaques that rupture cause blood clots to form that can block blood flow or break off and can cause a heart attack or stroke.

Many medical devices and therapeutic methods are known for the treatment of atherosclerotic disease. Probably the most common one is percutaneous transluminal angioplasty (PTA). PTA is based on Gruntzig's original concept of using a noncompliant balloon mounted on a double lumen catheter. One lumen allows the balloon catheter to be advanced over a guide wire and permits injection of contrast material, while the second lumen serves to inflate the balloon to a predetermined diameter. Briefly, a balloon-tipped catheter is inserted in a patient's artery, the balloon being deflated. The tip of the catheter is then advanced to the site of the atherosclerotic plaque to be dilated. The balloon is placed within/across the stenotic segment and subsequently inflated. As a result, the balloon "cracks" the atherosclerotic plaque and expands the vessel, thereby relieving the stenosis.

A device such as an intravascular stent can be a useful adjunct to PTA, particularly in the case of either acute or threatened closure after angioplasty. The use of PTA is, however, hampered by the observation that the treated blood vessel may suffer acute occlusion immediately after or within the initial hours after the dilation procedure. Another limitation encountered in PTA is called restenosis, i.e. re-narrowing of an artery after an initial successful angioplasty. This conditions arises typically during the first six months after angioplasty and is believed to be caused by proliferation and migration of vascular endothelial cells and/or by remodelling of the arterial wall.

Nevertheless, local sustained-release delivery systems may still offer the best way to treat certain medical conditions where high local concentrations and/or controlled delivery of the therapeutic agent is desired such that problems of systemic toxicity are essentially avoided. Conditions and diseases other than atherosclerosis are treatable with stents, catheters, cannulae and other devices partly or completely inserted into the vascular system, esophagus, trachea, colon, biliary tract, urinary tract, pancreas, uterus or other location within a body portion such as a passage, lumen or blood vessel of a human or veterinary patient.

It would be desirable to develop devices and methods for reliably delivering suitable therapeutic agents directly into a body portion during or following a medical procedure, so as to treat or prevent such conditions and diseases.

Metallic stents covered with a first composite layer of a polymer and of a therapeutically active substance coated with a second layer of fibrin are disclosed in Patent Application EP-A-0 701 802.

Known stent designs further include drug-impregnated polymer-coated metallic stents and biodegradable drug-eluting polymer stents coated with paclitaxel for the treatment of atherosclerosis (EP 0 711 158 Bl).

The invention of EP 0 747 069 is directed to implantable medical devices whereby the surface of the structure or at least in one part of the structure such as wells, holes, grooves, slots or the like are loaded with the therapeutic agent. An additional porous layer enables controlled release of the therapeutic agent.

EP 0 809 515 relates inter alia to a biodegradable stent with the therapeutic agent impregnated therein, i.e. in the stent material, and which is further coated with a biodegradable coating or with a porous or permeable non-biodegradable coating comprising a sustained release-dosage form of a therapeutic agent. This embodiment of the invention can provide a differential release rate of the therapeutic agent, i.e., there would be a faster release of the therapeutic agent from the coating followed by delayed release of the therapeutic agent that is impregnated in the stent matrix upon degradation of the stent matrix.

US 6,562,065 discloses an expandable medical device comprising a plurality of elongated beams, the plurality of elongated beams joined together to form a substantially cylindrical device wherein the elongated beams include a plurality of holes for containing a beneficial agent.

### SUMMARY OF THE INVENTION

The present invention provides an implantable device according to claim 1. Further preferred aspects of the invention are provided according to the dependent claims. In addition, the invention provides a method of treatment using such implantable devices.

In another aspect, the invention provides an implantable device according to claim 10. The invention is also directed to a device suitable for implantation in a living animal according to claim 23. Further, the invention provides a stent arrangement according to claim 24 and an implantable device according to claim 29. The device of the invention may comprise drug eluting fibres and there is provided a device according to claim 34.

In one aspect of the invention there is provided an implantable device as a delivery device for at least one therapeutic agent being composed of at least one type of base material comprising at least two types of reservoirs for at least one therapeutic agent whereby each type of reservoir independently provides identical or different release rates for the at least one therapeutic agent.

One further aspect of the invention involves the in vivo placement of implantable devices which are composed of at least one type of base material, e.g. of metallic, plastic or biodegradable material comprising at least two types of reservoirs for at least one therapeutic agent whereby each type of reservoir independently provides identical or different release rates for the at least one therapeutic agent.

Implantable devices which are composed of a combination of different types of base material, such as of a combination of metallic or plastic material with biodegradable material, comprising at least two types of reservoirs for at least one therapeutic agent whereby each type of reservoir independently provides identical or different release rates for the at least one therapeutic agent are also considered.

Briefly stated, the present invention provides therapeutic compositions, as well as methods and devices which utilize such compositions for the treatment of diseases and conditions where high local concentrations and/or controlled delivery of at least one therapeutic agent is desired such that problems of systemic toxicity are essentially avoided.

The term "therapeutic agent" is understood to include therapeutic and diagnostic agents such as, for example, drugs, vaccines, hormones, steroids, proteins, complexing agents, salts, chemical compounds, polymers, and the like. The term "therapeutic agent" is further understood to include any material that interacts with the tissues, cells, cell membranes, proteins and fluids of a human or an animal to improve the diagnosis, treatment or prevention of any physiologic or pathologic condition.

The therapeutic agent can include but is not limited to immunosuppressive agents, antitumor and/or chemotherapeutic agents, antimitotics, antiproliferatives, non-steroidal anti-inflammatory drugs, antimicrobials or antibiotics, growth factors and growth factor antagonists, thrombolytics, vasodilators, antihypertensive agents, antisecretory agents, antipolymerases, antiviral agents, photodynamic therapy agents, antibody targeted therapy agents, prodrugs, sex hormones, free radical scavengers, antioxidants, biologic agents, radiotherapeutic agents, radiopaque agents and radiolabelled agents.

Accordingly, it is preferred that the reservoirs of at least one therapeutic agent of the implantable device according to the invention comprises at least one therapeutic agent selected from the group of
1. cyclosporin, rapamycin, SDZ RAD or another immunosuppressive agent
2. taxol, or other anti-cancer chemotherapeutic agents
3. methotrexate or another antimetabolite or antiproliferative agent;
4. tamoxifen citrate;
5. dexamethasone, dexamethasone sodium phosphate, dexamethasone acetate or another dexamethasone derivative, or another antiinflammatory steroid or non-steroidal antiinflammatory agent;
6. an antiangiogenic agent (e.g. taxol, retinoic acid, anti-invasive factor, TNP-470, squalamine, plasminogen activator inhibitor-1 and -2 etc.)
7. colchicine or another antimitotic, or another microtubule inhibitor
8. smooth muscle migration and/or contraction inhibitors (e.g. cytochalasin B, C, and D) or another actin inhibitor
9. another growth factor antagonist; dopamine, bromocriptine mesylate, pergolide mesylate or another dopamine agonist
10. a growth hormone antagonist such as angiopeptin and angiogenin;
11. heparin, covalent heparin, or another thrombin inhibitor, hirudin, another antithrombogenic agent, or mixtures thereof;
12. urokinase, streptokinase, a tissue plasminogen activator, or another thrombolytic agent, or mixtures thereof;
13. a fibrinolytic agent;
14. a vasospasm inhibitor;
15. a protein kinase inhibitor (e.g stauroporin)
16. a calcium channel blocker,
17. a nitrate, nitric oxide, a nitric oxide promoter or another vasodilator;
18. an antiatherosclerotic agent
19. a antihypertensive agent;
20. a antiplatelet agent;
21. an antihistamic and/or antiallergic agent (e.g. terfenadine)
22. an antimicrobial agent or antibiotics (e.g. penicillin, streptomycin, cephalosporin, vancomycin, erythromycin, polymyxin, rifampycin, tetracycline, chloramphenicol etc.)
23. deoxyribonucleic acid, an antisense nucleotide or another agent for molecular genetic intervention;
24. <60>Co (5.3 year half life), <192>Ir (73.8 days), <32>P (14.3 days), <111>In (68 hours), <90>Y (64 hours), <99m>Tc (6 hours) or another radiotherapeutic agent; iodine-containing compounds, barium-containing compounds, gold, tantalum, platinum, tungsten or another heavy metal functioning as a radiopaque agent;
25. a peptide, a protein, an enzyme, an extracellular matrix component, a cellular component or another biologic agent;
26. a free radical scavenger, iron chelator or antioxidant;
27. progesteron, estrogen or another sex hormone;
28. antiviral agents, AZT or other antipolymerases; acyclovir, famciclovir, rimantadine hydrochloride, ganciclovir sodium, Norvir, Crixivan,
29. gene therapy agents;
or analogs or derivatives or functional equivalents thereof.

Within a preferred embodiment of the invention, the implantable device is intended for use in the vascular system (booth arteries and veins), at least of the reservoir preferably comprises cyclosporin, rapamycin, SDZ RAD or another immunosuppressive agent, taxol or the derivatives thereof, or other anti-cancer chemotherapeutic agents, heparin or another thrombin inhibitor or antiplatelet agent, hirudin, another antithrombogenic agent, or mixtures thereof; urokinase, streptokinase, a tissue plasminogen activator, or another thrombolytic agent, or mixtures thereof; dexamethasone, dexamethasone sodium phosphate, dexamethasone acetate or another dexamethasone derivative, or another antiinflammatory steroid or non-steroidal antiinflammatory agent. Representative example of suitable sites include coronal, iliac and renal arteries, and the superior vena cava.

Within a very preferred embodiment of the invention, the implantable device is intended for the treatment of cancer, atherosclerosis and angiogenesis-dependent diseases.

Representative but not limiting examples which may be treated utilizing the compositions and implantable devices described herein include metastastic and non-metastatic tumors (which can be derived from virtually any tissue, the most common being from lung, breast, melanoma, kidney, and gastrointestinal tract tumors), lymphomas (e.g.,Hodgkin's and Non-Hodgkin's Lymphoma including numerous subtypes, both primary and secondary); sarcomas (malignant tumor of soft tissue such as muscles, tendons, fibrous tissues, fat, blood vessels and nerves); brain tumors (such as astrocytoma, ependymoma; glioblastoma), neuron tumors (e.g. medulloblastoma, neuroblastoma); and tumors of nerve sheath cells (e.g.
Schwannoma and Neurofibroma).

Tumors that grow larger than about one to two millimeters in size will require new blood vessel growth to supply oxygen and nutrients to the cells and carry waste away. Thus, within one aspect of the invention, growth of vascular endothelial cells involved in the genesis of new blood vessels will be specifically suppressed when treated treated utilizing the compositions and implantable devices described herein. As a result, tumors deprived of vascularization will no longer grow.

Yet in another aspect of the invention, angiogenesis-dependent diseases characterized by the abnormal growth of blood vessels may also be treated with the anti-angiogenic compositions to reduce the growth of new blood vessels.

Representative but not limiting examples of such angiogenesis-dependent diseases include psoriasis, hypertrophic scarring and keloids, delayed wound healing, surgical and vascular adhesions, neovascular diseases of the eye, proliferative diabetic retinopathy, rheumatoid arthritis, arteriovenous malformations (discussed above), atherosclerotic plaques, hemophilic joints, nonunion fractures and the Osler-Weber syndrome.

Within various embodiments of the invention, implantable devices are provided for eliminating biliary obstructions comprising inserting a biliary stent into a biliary passageway; for eliminating urethral obstructions, comprising inserting a urethral stent into a urethra; for eliminating esophageal obstructions, comprising inserting an esophageal stent into an esophagus; and for eliminating tracheal/bronchial obstructions, comprising inserting a tracheal/bronchial stent into the trachea or bronchi; for eliminating fallopian tube obstructions, comprising inserting a fallopian tube stent into the fallopian tube; for eliminating ureteric obstructions by inserting an ureteric stent into the uterus; for eliminating Eustachian tube obstructions, comprising inserting a Eustachian tube stent into the Eustachian tube; for eliminating pancreatic obstructions comprising inserting a pancreatic stent into the pancreas.

Thus, within a further preferred embodiment of the invention, the implantable device is intended for use in the biliary tract such that the biliary obstruction is eliminated. Most commonly, the biliary system which drains bile from the liver into the duodenum is most often obstructed by (1) a tumor which invades the biliary tract (e.g., pancreatic carcinoma), (2) a tumor composed of biliary tract cells (cholangiocarcinoma), or (3) a tumor which exerts extrinsic pressure and compresses the biliary tract (e.g., enlarged lymph nodes). Both primary biliary tumors, as well as other tumors which cause compression of the biliary system may be treated utilizing the stents described herein.

Within a further preferred embodiment of the invention, the implantable device is intended for use in the esophagus such that the esophagus obstruction is eliminated, e.g. in the case of cancer in the esophagus or invasion by benign or malign cancer cells arising in adjacent organs (e.g. cancer of the lung or stomach).

Within a further preferred embodiment of the invention, the implantable device is intended for use in the urinary tract such that urethral obstruction are eliminated, e.g. in the case of hypertrophy of the prostate.

In still another embodiment of the present invention, implantable devices other than stents include, without limitation, hip joints, artificial heart valves, pace maker, catheters, ophthalmic lenses, orthopedic or dental prostheses are considered to be utilized according to the invention.

For example, it has been demonstrated that calcium phosphate coatings on metal implants (e.g. hip stems) allow a rapid bone apposition due to their osteoconductive property, as compared with bare implants. As a result from the contact with body fluids, a thin layer of biological hydroxyl carbonated apatite (HCA) is formed on the surface of some implants followed by living bone tissue is directly apposited to this HCA layer. The direct bone apposition onto and/or growth into the implant surface significantly improves the healing process and long term results. Thus, implantable devices according to the invention can comprise at least two types of reservoirs (on of which, for example, is a calcium phosphate coating) for at least one therapeutic agent which can be used in a variety of medical applications such as surgery, bone-replacement, prosthodontics, dental roots, crowns, orthopedic joints, etc.

In still another embodiment of the present invention, implantable devices can be used to treat antimicrobial resistance. For example, respiratory infections, HIV/AIDS, diarrhoeal diseases, tuberculosis and malaria are the leading causes among the infectious diseases. Resistance to first-line therapeutic agents, however, has been observed in all these diseases. Moreover, drug resistance is an especially difficult problem for hospitals because they harbor critically ill patients who are more vulnerable to infections than the general population and therefore require more antibiotics. Thus, implantable devices according the invention can comprise at least two types of reservoirs for at least one therapeutic agent which can be used to treat antimircrobial resistance.

In its simplest form, the invention is directed to an implantable medical device comprising a structure (e.g. stents) adapted for introduction into the esophagus, trachea, colon, biliary tract, urinary tract, vascular system or other lumens of a body portion such as passage or blood vessel in a living human or veterinary patient, the structure being composed of at least one type of base material, e.g. of metallic, plastic or biodegradable material comprising at least 2 types of reservoirs for at least one therapeutic agent whereby each type of reservoir independently provides identical or different release rates for the at least one therapeutic agent.

Generally, stents are inserted in a similar fashion regardless of the site or the disease being treated. Typically, stents are capable of being compressed, so that they can be inserted through tiny cavities via small catheters, and then expanded to a larger diameter once they are at the desired location. Once expanded, the stent physically forces the walls of the passageway apart and holds it open. As such, they are capable of insertion via a small opening, and yet are still able to hold open a large diameter cavity or passageway. The stent may be balloon expandable, self-expanding or implanted and expanded by a change in temperature using so-called memory-metal alloys e.g.. nitinol.

Within one aspect of the present invention, stents are provided comprising a generally tubular structure, the stent being loaded with one or more therapeutic compositions. Briefly, a stent is a scaffolding, usually cylindrical in shape, that may be inserted into a body lumen (e.g. blood vessel, biliary tract), which has been narrowed by a disease process (e.g., stenosis, tumor growth) in order to prevent closure or reclosure of the body lumen.

Within other aspects of the present invention, implantable devices are provided for expanding the lumen of a body lumen, comprising inserting a stent into the lumen, the stent having a generally tubular structure, the stent structure and/or the surface being loaded with a therapeutic composition.

The implantable devices of the invention are made of at least one type of base material, such as metallic, plastic or biodegradable material. A combination of at least two different types of base material (e.g. metallic/biodegradable or plastic/biodegradable) is contemplated or as well as a combination of three different types of base material, i.e. metallic/ biodegradable/plastic.

Accordingly, the base material can include at least one of stainless steel, tantalum, titanium, nitinol, gold, platinum, chromium, iridium, silver, tungsten, cobalt or another biocompatible metal, or alloys of any of these;
cellulose acetate, cellulose nitrate, polylactic acid, polyglycolic acid or copolymers thereof, carbon or carbon fiber; a polyanhydride, polycaprolactone, polyhydroxybutyrate valerate or another biodegradable polymer, or mixtures or copolymers of these; silicone, polyethylene teraphthalate, polyurethane, polyamide, polyester, polyorthoester, polyanhydride, polyether sulfone, polycarbonate, polypropylene, high molecular weight polyethylene, polytetrafluoroethylene, or another biocompatible polymeric material, or mixtures or copolymers of these; a protein, an extracellular matrix component, collagen, fibrin, starch or another biologic agent; or a suitable mixture of any of these.

A cobalt/chromium alloy is particularly useful as the base material when the structure is configured as a vascular stent.

Implantable devices may be loaded with the therapeutic composition(s) of the present invention using a combination of at least two different reservoirs for the at least one therapeutic agent is selected from
(a) directly affixing an therapeutic composition to the surface structure of the implantable device resulting in a biodegradable coating or in a porous or permeable non-biodegradable coating (completely or partly);
(b) filling the therapeutic agent into the opening(s) of the implantable device (e.g. hole, well, groove, slot and the like;
   and/or
(c) impregnating the base material of the implantable device with the therapeutic composition.

For example, the implantable device according to the invention can comprise the combination of filled openings and a coated surface; or filled openings and an impregnated base material; or a coated surface and an impregnated base material.

In a particular embodiment, the implantable device comprises reservoirs for at least one therapeutic agent based on a combination of filled openings, coated surface areas and impregnated base material.

The holes, wells, grooves, slot and the like may be formed in the surface of the implantable device by a variety of techniques. For example, such techniques include drilling or cutting, use of lasers, electron-beam machining and the like or employing other procedures know to the person skilled in the art such as etching the desired apertures. Coating of the surface and impregnation of the base material can be achieved by any conventional coating technique known to the skilled person (e.g. dipping, spraying, electrochemical deposition) suitable for the therapeutic agent to keep its therapeutic activity.

The combination of different types of reservoirs for at least one therapeutic agent allows to independently configure desired controlled release patterns of the therapeutic agent(s) in view of the disease to be treated, the disease state, the body lumen, the type of therapeutic agent (e.g. hydrophilic, lipophilic, biologics, small molecules etc.), desired concentration of at least one therapeutic agent, favorable combinations of therapeutic agents, desired kinetic release patterns (zero order pulsatile, increasing, decreasing, sinusoidal etc.).

As a result, each type of reservoir for at least one therapeutic agent can be independently designed to provide tailored and optimised dosing of therapeutic agent(s). The combination of at least two different types of reservoirs for at least one therapeutic agent confers customized release kinetics for the targeted delivery of the agent(s) at various sites of the body, thus reducing/eliminating unwanted side effects such as systemic toxicity.

As the coating is necessarily thin and the surface area is relative short, the therapeutic agent tends to have a short diffusion path to discharge resulting in a burst release on the agent. The drug reservoir in the openings and the impregnated base material can confer, however (but do not have to) delayed release of the therapeutic agent upon degradation of the biodegradable polymer.

However, surface coating which confers delayed release is also contemplated. Further, the stent may be coated with two or more layers comprising the same or different therapeutic agents housed in the same or different coating material. These additional layers can be placed directly on top of each other or can be separated by additional porous or permeable non-biodegradable layers.

An implantable device comprising a combination of a metallic part and a biodegradable part is contemplated. Further, only part of the device needs to be coated. Moreover, different parts of the device can be loaded with the same or different therapeutic agent(s). It is also contemplated that different regions or sides of the same part of the device can be loaded with the therapeutic agent(s).

In still another embodiment of the present invention, the base material of the implantable device is made (at least partly) of a material such that detection of the implantable device after insertion into the body lumen is facilitated. In such an embodiment, for example in the form of a stent. the stent could comprise a central stainless steel region with end regions formed of a metal providing a higher or lower signal in a NMR or CT scanner.

In still another embodiment of the present invention, the base material of the implantable device is made (at least partly) of a material such that after insertion into the body lumen, the healing process within the body lumen can be better observed. For example, the base material can partly be made of translucent material and/or biodegradable material. For example, after degradation of the biodegradable part of a stent placed into the vascular system, monitoring of healing effect of the therapeutic agent(s) on the vascular endothelia cells is facilitated.

A combination of all degrees of freedom allows loading of the reservoirs for at least one therapeutic agent of the implantable device according to the invention in a programmable manner specifically adapted to the situation/phase/requirement during the medicinal treatment.

For example, the therapeutic agent A can be released in an initial burst based on the surface coating resulting in a locally high concentration of the agent following by a controlled retarded release (ideally over a period of weeks to months) conferred by the reservoir(s) in the filled openings and/or impregnated base material.

In another scenario, the therapeutic agent A can be released in an initial burst based on the surface coating following by a controlled retarded release of therapeutic agent B conferred by the reservoir(s) in the filled openings and/or impregnated base material.

A further application of the implantable device according to the invention could be the combination of at least two different therapeutic agents each having reservoirs which confer different types of release patterns (e.g. agent A: pulsatile/decreasing; agent B: sinusoidal).

### BRIEF DESCRIPTION OF THE DRAWINGS

The invention will now be described, by way of example only, with reference to the drawings in which:
- Fig.1: shows a cross section of a coated stent;
- Fig. 2: shows a schematic illustration of the coated stent of Fig. 1;
- Fig 2a: shows a cross section of a self expanding coated stent;
- Fig. 3: shows a cross-section of a stent having two coating layers;
- Fig. 4: shows a schematic illustration of a stent having island coating regions;
- Fig. 5: is a schematic illustration of a multiregion stent;
- Fig. 6: is a further schematic illustration of a multiregion stent; and
- Fig. 7: is a schematic illustration of a multiregion stent having an intermediate structure.

### DESCRIPTION OF THE PREFERRED EMBODIMENTS

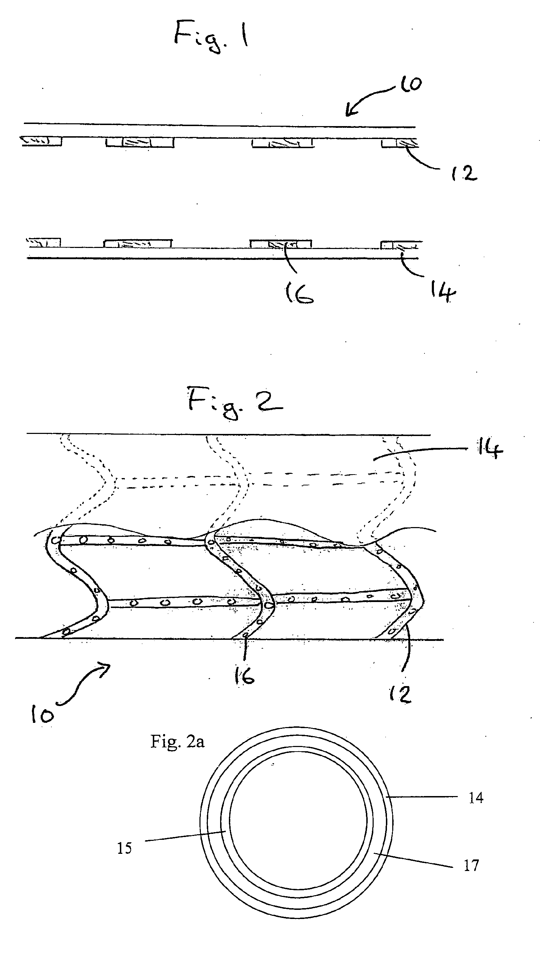

Referring to Fig. 1, there is shown a cross-section of a stent 10. The stent 10 comprises a metallic scaffolding formed of an arrangement of stainless steel struts 12 coated with a polymer layer 14. The struts 12 include therein through-holes 16 which are filled with a drug-eluting biodegradable polymer reservoirs fabricated in a manner as described in EP 0 747 069. The polymer layer 14 is also biodegradable and drug-eluting.

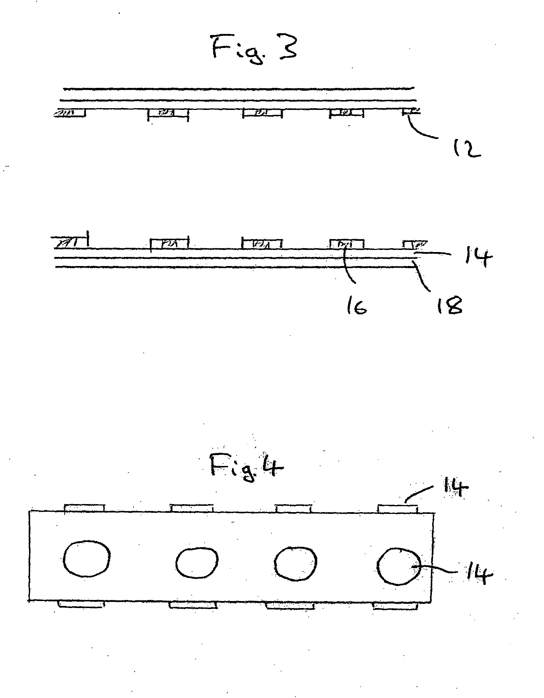

The stent 10 shown in Fig. 1 is shown in schematic form in Fig. 2 in which for ease of understanding the polymer layer 14 is shown only partially to reveal the scaffolding structure underneath of the struts 12 including polymer filled through-holes 16. The details of the scaffolding structure are not important for the understanding of the invention. As shown, the scaffolding is similar to the arrangement illustrated in EP-A-0 540 290 but could take other forms. Suitable stent arrangements are described in the "Handbook of Coronary Stents", second edition, Rotterdam Thoraxcenter Group, 1998, Mosby. Although the polymer layer 14 is shown as being a continuous covering, in practice, the layer could cover only the underlying scaffolding, either wholly or partly. The polymer may be the same as that filling the through holes 16, in which case a bond to these regions would generally be formed, or a different polymer may be used to provide a particular drug-elution rate profile.

If the polymer layer 14 is to provide a continuous surface such as that shown in Fig. 2, this could be obtained by attaching a sheet of polymer to the surface of the drug impregnated stent by wrapping the stent in the sheet and joining two sides of the sheet together. By exerting pressure on the sheet, at a slightly elevated temperature, a bond will form with the polymer within the through holes 16. If only the underlying scaffolding is to be covered, this could be achieved by dipping the drug in a polymer/drug solution so as to coat the exposed metal with the polymer. The solvent is then evaporated to leave the drug containing polymer coating.

For certain applications, in which a metal stent is covered with a biodegradable outer covering, it would be beneficial if the metal stent could be arranged to expand after the covering has been absorbed into the body in order to provide an optimal support of the lumen wall. If the metal stent is of the self expanding type, the biodegradable covering would have the effect of restraining the expansion of the stent within the patient. If the expansion is, however, balloon in a manner to cause plastic deformation of the outer covering within the patients lumen, this will enable the inner self-expanding stent to be inserted into position within the patient and once the degradable covering has been absorbed, a continued effective support will be achieved. A further mechanism of enabling a long term good supporting action once the degradable coating has been withdrawn would be to provide a plating on the inside of the stent of an oxide forming material, such as magnesium. As the magnesium oxidizes, the internal stresses resulting will cause the overlying metal stent to expand slightly, compensating for the absorption of the biodegradable covering. The inner surface of the stent may also comprise a biodegradable drug-eluting coating. Such an arrangement is shown in Fig. 2a in which a metal self expanding stent17 is covered with a biodegradable drug-eluting coating 14. On the inner surface of the stent 17, a further biodegradable drug eluting coating 15 is provided. The drugs eluted from the coatings 14 and 15 may be the same or may be different. The stent 17 may also be drug eluting.

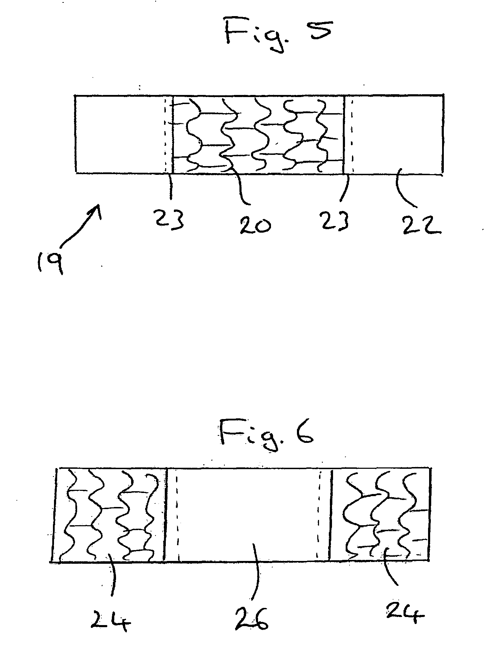

Fig. 3 shows a similar arrangement except that the stent includes two polymer layers 14 and 18 containing different therapeutic compositions. As shown in Fig. 4, the polymer layers need not be continuous but may be in the form of discrete islands on the underlying metal scaffolding.

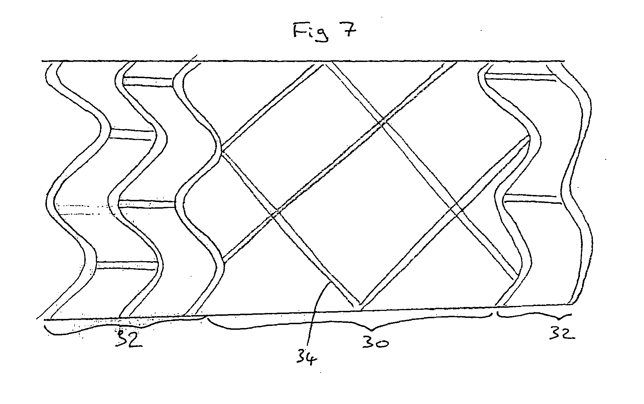

A further aspect of the invention is illustrated in Fig. 5. This figure shows a stent 19 comprised of a central metallic region 20 and two polymer end regions 22. The metallic region 20 has a conventional scaffolding arrangement with drug eluting reservoirs therein. The polymer end regions are also drug eluting but are biodegradable. There is a region of overlap 23 in which the polymer end regions 23 overlap the underlying metallic region. In this overlap region, there are through-holes through the metal which are filled with the polymer forming the end region 22, providing a strong bond between the two regions. Accordingly, after insertion into a patient, a locally high dose of the therapeutic medicament will be provided in the region of the desired treatment and after the polymer has been adsorbed into the patient, only the relatively small region of the metallic stent would remain. This arrangement thus enables a relatively higher concentration of medicament to be provided in the desired locality than would be provided by the metallic part of the stent alone.

An alternative arrangement to that shown in Fig. 5 is shown in Fig. 6. In this arrangement, there is a central biodegradable polymer region 26 and two metallic end regions 24. If desired, the central polymer region 26 can be formed over a metal support, as shown in Fig. 7. In this figure, a central region 30 has a metallic structure formed of struts 34 connecting to end regions 32. During manufacture, a drug-eluting polymer is cast over the struts 34 to form the polymer region 26 (not shown in Fig. 7). The struts 34 serve to provide a physical permanent connection between the end regions 32 but have a structure which is significantly more open than the metallic end region structure. Accordingly, after insertion into a patient, the central region is significantly more transparent to X-rays, for example coming from a CT scanner. As the reader will appreciate, the central region illustrated in Fig. 7 is not drawn to scale.

If, for example, the implantable device is a stent comprising three tubular sections, a central metal tubular section and a biodegradable tubular section attached at each end, this could be manufactured by forming the central metal region in a conventional fashion known to those skilled in the art of stent manufacture to provide a scaffolding structure. This could then be loaded with a drug-eluting agent, for example by filling apertures in the metal tube with a drug containing biodegradable polymer. Drug loaded biodegradable polymer tubular sections could then be attached to this metal portion using a body-compatible adhesive such as a silicone adhesive. The combined structure can then receive a full or partial coating of a biodegradable, drug eluting polymer.

Alternatively, the biodegradable polymer section could be attached to the metal portion by directly molding the polymer onto an end region of the metal. In this embodiment, the end portion of the metal region would preferably include holes drilled through the metal such that in the molding process, the polymer could enter the holes and once solidified form an attachment mechanism.

Of course, the stent could have a configuration of a central biodegradable region with non-degradable end regions attached thereto. In such an arrangement, it would be preferable for the central region to include a minimal metallic support structure to maintain the two metallic end regions in a given spatial relationship. Such an arrangement could be fabricated by cutting a pattern from a stainless steel tube such that a central region has a much lower metallic area compared with two end regions. The central region or the central region and the end regions could then receive a polymer molding.

Although in Figs 5 and 6, each polymer region is shown as being continuous, it may be desirable to provide a structure in which the polymer region is in the form of windows in the wall of an otherwise conventional metal stent. In the region of the windows, the scaffolding effect normally provided by metal struts is provided by the polymer. In such an arrangement, since the metal scaffolding is effectively continuous, there would be no requirement to have metal struts in the polymer reigon although this may be desirable to provide a desirable location and restraining capability to hold the polymer regions in place.

In addition to a stent having a metal central region and one or more polymer biodegradable end regions, a stent could be envisaged in which all regions are metallic. For example, a central region could be fabricated from stainless steel to provide long term support whilst one or both end regions could have attached thereto a stent region fabricated from a biodegradable metal alloy such as a magnesium alloy or other adsorbable metal as described in EP-A-0 966 979, incorporated herein by reference. Although the bonding of dissimilar metals is potentially complicated, techniques do exist, for example vacuum welding (especially electron beam welding) or gluing. It may be beneficial to include a plating layer on the stainless steel region to improve compatibility with the biodegradable metal region which could be dimensioned to fit over or within the stainless steel region. Diffusion bonding under pressure could provide a suitable joining mechanism. Again, the metal regions could include drug eluting polymer filled reservoirs and the whole or part of the structure could be coated with a drug-eluting polymer.

Recently, the use of nanofibers has been suggested for the release of NO in a controlled manner to tissues and organs, for example in WO 01/26702. Such nanofibers as described therein could be incorporated into the devices of the present invention. As an example, the fibers could be used to weave a fabric sleeve which could cover the stent structure, possible together with a biodegradable polymer matrix. Alternatively, a sleeve of such woven fibers could be used to connect to end metallic end regions. Additionally, short nanofiber lengths could be mixed with a polymer solution and the resulting mixture used to form a polymer/fibre composite structure.

Although the preceding description has concentrated on the drug-eluting possibilities for device construction, the ability to combine different materials in a single structure can also provide further benefits. One problem with existing stent designs is that when inserted into a patient, it becomes difficult to monitor the vessel in the region of the stent because the observation signal generated by the stent material is too high. Accordingly, it would be desirable to have a stent structure with a greater transparency for use in NMR or CT scanners. Such a structure can be obtained by filling voids in a relatively open stainless steel stent structure with a biodegradable polymer. After insertion into the patient, the polymer helps to support the vessel wall whilst at the same time allowing the physician to monitor the position of the stent and its local effect using conventional scanning technology. Later, as the support requirement reduces, the polymer can biodegrade. If this decrease in support capability is undesirable, a non-biodegradable polymer could be used. Such an arrangement would be similar to that shown in Figs. 5-7 but without any requirement for the polymer regions to be drug-eluting although of course this may be desirable.

## Claims

1. An implantable device as a delivery device for at least one therapeutic agent being composed of at least one type of base material comprising at least two types of reservoirs for at least one therapeutic agent whereby each type of reservoir independently provides identical or different release rates for the at least one therapeutic agent.

2. An implantable device according to claim 1 **characterized in that** the base material is selected from the group of metallic, plastic and/or biodegradable materials.

3. An implantable device according to claim 2 **characterized in that** the implantable device is made of a combination of a metallic and a biodegradable material.

4. An implantable device according to claim 2 **characterized in that** the implantable device is made of a combination of a plastic and a biodegradable material.

5. An implantable device according to one of claims 1 to 4 **characterized in that** the base material of the implantable device is made at least partly of a material such that detection of the implantable device after insertion into the body lumen is facilitated.

6. An implantable device according to one of claims 1 to 4 **characterized in that** the base material of the implantable device is made at least partly of a material such that after insertion into the body lumen, the healing process within the body lumen can be better observed.

7. An implantable device according to one of claims 1 to 4 **characterized in that** the metallic base material is selected from at least one of tantalum, titanium, gold, platinum, chromium, iridium, silver, tungsten, cobalt or alloys of any of these or stainless steel or nitinol or another biocompatible metal.

8. An implantable device according to claim 7 **characterized in that** the metallic base material is a cobalt/chromium alloy.

9. An implantable device according to claims 1 to 4 **characterized in that** the plastic and/or biodegradable base material is selected from at one of cellulose acetate, cellulose nitrate, polylactic acid, polyglycolic acid or copolymers thereof, carbon or carbon fiber; a polyanhydride, polycaprolactone, polyhydroxybutyrate valerate or another biodegradable polymer, or mixtures or copolymers of these; silicone, polyethylene teraphthalate, polyurethane, polyamide, polyester, polyorthoester, polyanhydride, polyether sulfone, polycarbonate, polypropylene, high molecular weight polyethylene, polytetrafluoroethylene, or another biocompatible polymeric material, or mixtures or copolymers of these; a protein, an extracellular matrix component, collagen, fibrin, starch or another biologic agent; or a suitable mixture of any of these.

10. An implantable device **characterized in that** the implantable device comprises at least one therapeutic composition within at least two different reservoirs for the at least one therapeutic agent wherein
the therapeutic composition is directly affixed to a surface structure of the implantable device as a biodegradable or porous or permeable non-biodegradable coating;
the therapeutic composition is contained within openings in the implantable device; and/or
the therapeutic composition is impregnated in the base material of the implantable device.

11. An implantable device according to claim 10 **characterized in that** a combination of filled openings and a coated surface is used as reservoirs for the at least one therapeutic agent.

12. An implantable device according to claim 10 **characterized in that** the device comprises a combination of filled openings and an impregnated base material.

13. An implantable device according to claim 10 **characterized in that** the device comprises a combination of a coated surface and an impregnated base material.

14. An implantable device according to claim 10 **characterized in that** the device comprises a combination of filled openings, coated surface areas and impregnated base material.

15. An implantable device according to one of claims 1 to 14 **characterized in that** the reservoirs for the at least one therapeutic agent comprises at least one therapeutic agent selected from the group of immunosuppressive agents, antitumor and/or chemotherapeutic agents, antimitotics, antiproliferatives, non-steroidal anti-inflammatory drugs, antimicrobials or antibiotics, growth factors and growth factor antagonists, thrombolytics, vasodilators, antihypertensive agents, antisecretory agents, antipolymerases, antiviral agents, photodynamic therapy agents, antibody targeted therapy agents, prodrugs, sex hormones, free radical scavengers, antioxidants, biologic agents, radiotherapeutic agents, radiopaque agents and radiolabelled agents.

16. An implantable device according to claim 15 **characterized in that** the reservoirs for the at least one therapeutic agent comprises at least one therapeutic agent selected from the group of
cyclosporin, rapamycin, SDZ RAD or another immunosuppressive agent; taxol, or other anti-cancer chemotherapeutic agents; methotrexate or another antimetabolite or antiproliferative agent; tamoxifen citrate; dexamethasone, dexamethasone sodium phosphate, dexamethasone acetate or another dexamethasone derivative, or another antiinflammatory steroid or non-steroidal antiinflammatory agent; an antiangiogenic agent (e.g. taxol, retinoic acid, anti-invasive factor, TNP-470, squalamine, plasminogen activator inhibitor-1 and -2 etc.); colchicine or another antimitotic, or another microtubule inhibitor; smooth muscle migration and/or contraction inhibitors (e.g. cytochalasin B, C, and D) or another actin inhibitor; another growth factor antagonist; dopamine, bromocriptine mesylate, pergolide mesylate or another dopamine agonist; a growth hormone antagonist such as angiopeptin and angiogenin; heparin, covalent heparin, or another thrombin inhibitor, hirudin, another antithrombogenic agent, or mixtures thereof; urokinase, streptokinase, a tissue plasminogen activator, or another thrombolytic agent, or mixtures thereof; a fibrinolytic agent; a vasospasm inhibitor; a protein kinase inhibitor (e.g stauroporin); a calcium channel blocker; a nitrate, nitric oxide, a nitric oxide promoter or another vasodilator; an antiatherosclerotic agent; an antihypertensive agent; an antiplatelet agent; an antihistamic and/or antiallergic agent (e.g. terfenadine); an antimicrobial agent or antibiotics (e.g. penicillin, streptomycin, cephalosporin, vancomycin, erythromycin, polymyxin, rifampycin, tetracycline, chloramphenicol etc.); deoxyribonucleic acid, an antisense nucleotide or another agent for molecular genetic intervention; <60>Co, <192>Ir, <32>P, <111>In, <90>Y, <99m>Tc or another radiotherapeutic agent; iodine-containing compounds, barium-containing compounds, gold, tantalum, platinum, tungsten or another heavy metal functioning as a radiopaque agent; a peptide, a protein, an enzyme, an extracellular matrix component, a cellular component or another biologic agent; a free radical scavenger, iron chelator or antioxidant; progesterone, estrogen or another sex hormone; an antiviral agent, AZT or other antipolymerases; acyclovir, famciclovir, rimantadine hydrochloride, ganciclovir sodium, Norvir or Crixivan; gene therapy agents; or analogs or derivatives or functional equivalents thereof.

17. An implantable device according to one of claims 1 to 16 **characterized in that** the at least two types of reservoirs for at least one therapeutic agent allows to independently configure desired controlled release patterns of the therapeutic agent(s) in view of the disease to be treated, the disease state, the body lumen, the type of therapeutic agent (e.g. hydrophilic, lipophilic, biologics, small molecules etc.), desired concentration of at least one therapeutic agent, favorable combinations of therapeutic agents, desired kinetic release patterns (zero order pulsatile, increasing, decreasing, sinusoidal etc.).

18. An implantable device according to one of claims 1 to 17 **characterized in that** the implantable device is a stent, a hip joint, an artificial heart valve, a pace maker, a catheter, an ophthalmic lens, an orthopedic prosthesis or a dental prosthesis.

19. An implantable device according to claim 18 **characterized in that** the implantable device is a stent adapted for introduction into the esophagus, trachea, colon, biliary tract, urinary tract, vascular system or other lumens of a body portion such as passage, lumen or blood vessel in a living human or animal.

20. Use of composition loaded on an implantable device as a delivery device for at least one therapeutic agent being composed of at least one type of base material comprising at least two types of reservoirs for at least one therapeutic agent whereby each type of reservoir independently provides identical or different release rates for the at least one therapeutic agent for the treatment of cancer, atherosclerosis and angiogenesis-dependent diseases.

21. Use of composition loaded on an implantable device as a delivery device for at least one therapeutic agent being composed of at least one type of base material comprising at least two types of reservoirs for at least one therapeutic agent whereby each type of reservoir independently provides identical or different release rates for the at least one therapeutic agent for use in medical applications such surgery, bone-replacement, prosthodontics, dental roots, crowns and orthopedic joints.

22. Use of composition loaded on an implantable device as a delivery device for at least one therapeutic agent being composed of at least one type of base material comprising at least two types of reservoirs for at least one therapeutic agent whereby each type of reservoir independently provides identical or different release rates for the at least one therapeutic agent for the treatment of antimircrobial resistance.

23. A device suitable for implantation in a living animal comprising a first region and at least a second region wherein the first region is of a material having a first response characteristic to electromagnetic radiation and the second region has a second response characteristic to electromagnetic radiation different to said first response characteristic,
**characterized in that** the second region is incorporated into the structure of the device to provide, at least temporarily, substantially the same physical properties as if it were fabricated of the first material but because of its differing response characteristic to electromagnetic radiation provides an improved image generating capability in relation to an imaging technique used for internally imaging the device in the living animal compared with if the second region was fabricated of the first material.

24. A stent for implantation in a lumen of a patient **characterized in that** said stent comprises a plurality of first supporting regions having metal lumen wall supporting means and at least one second supporting region within or between the metal supporting regions, the second supporting region comprising a structural polymer lumen wall supporting means.

25. A stent according to claim 24, **characterized in that** the at least one second supporting region comprises an arrangement of metal struts joined to metal lumen wall supporting means, the metal struts of the polymer supporting region having a projected surface area per unit surface area of the stent ratio substantially less than a projected surface area to unit surface area of the stent ratio of the metal lumen wall supporting means.

26. A stent according to claim 24 or claim 25, wherein the structural polymer is biodegradable.

27. A stent according to any one of claims 24 to 26 wherein the structural polymer is drug-eluting.

28. A stent according to any one of claims 24 to 27 wherein the metal lumen wall supporting means are drug eluting.

29. An impantable device for implantation in a patient **characterized** that the device comprises a metallic structure having windows therein filled by a polymer.

30. An implantable device according to claim 29 wherein said polymer is a biodegradable polymer.

31. An implantable device according to claim 29 or claim 30 wherein the polymer is a drug-eluting polymer.

32. An implantable device according to one of claims 29 to 31 wherein said metallic structure incorporates drug-eluting regions.

33. An implantable device according to one of claims 29 to 32 wherein said device is a stent.

34. A device for implantation in the body of an animal **characterized** that it includes a composite material comprising a polymer and drug eluting fibers.

35. A device according to claim 34 wherein said drug eluting fibers are woven.

36. A stent **characterized in that** it comprises a self-expanding support structure and a biodegradable drug-eluting layer on an outer surface thereof wherein the self expanding support structure expands after insertion into a patient as the coating is adsorbed into the patient.

37. A stent according to claim 36 wherein the support structure is a self expanding metal support structure.

38. A stent according to claim 36 or claim 37 wherein the self-expanding support structure includes drug-eluting means.

39. A stent according to one of claims 36 to 38 wherein the stent further comprises a drug eleuting layer on an inner surface of the support structure.
